# EUROPÄISCHE PATENTSCHRIFT

(11) **EP 0 350 686 B1**
(45) Veröffentlichungstag und Bekanntmachung des Hinweises auf die Patenterteilung: **29.09.1993**
(21) Anmeldenummer: 89111529.7
(22) Anmeldetag: 24.06.1989
(51) Int. Cl.: C07C 69/675, C07C 67/31

(54) **Verfahren zur Herstellung von 6-Hydroxycapronsäureestern**
Process for the preparation of 6-hydroxycapronic-acid esters
Procédé de fabrication d'esters de l'acide 6-hydroxycaproique

(30) Priorität: 13.07.1988 DE 3823695
(43) Veröffentlichungstag der Anmeldung: 17.01.1990
(73) Patentinhaber: BASF Aktiengesellschaft, 67063 Ludwigshafen (DE)
(72) Erfinder: Richter, Wolfgang, Dr., D-6706 Wachenheim (DE); Harder, Wolfgang, Dr., D-6940 Weinheim (DE); Fischer, Rolf, Dr., D-6900 Heidelberg (DE); Vagt, Uwe, Dr., D-6720 Speyer (DE)

(56) Entgegenhaltungen:
- US-A- 3 189 619
- US-A- 3 253 018

## Beschreibung

Aus der US-Patentschrift 3 253 018 ist ein verfahren zur Herstellung von 6-Hydroxycapronsäuremethylester durch Hydrieren einer weniger als 10 gew.%igen Lösung von 5-Formylvaleriansäuremethylester in Diethylether in Gegenwart eines Platinoxidkatalysators bei Raumtemperatur unter einem Druck von 3 bis 4 bar bekannt. Ein solches verfahren eignet sich nicht für die technische Durchführung, da erhebliche Mengen an Lösungsmittel zurückgewonnen und wieder gereinigt werden müssen.

Nach einem anderen aus der US-Patentschrift 3 189 619 bekannten Verfahren werden 5-Formylvaleriansäureester in Gegenwart von Nickelkatalysatoren bei einer Temperatur von mindestens 150°C und unter einem Druck von über 175 bar Wasserstoff zu 6-Hydroxycapronsäureestern hydriert. Unter den angewandten Hydrierbedingungen erhält man jedoch erhebliche Mengen an Hochsiedern wie di-, tri- und polymeren Hydroxycapronsäureestern.

Es war deshalb die technische Aufgabe gestellt, bei der Hydrierung von 5-Formylvaleriansäureestern zu 6-Hydroxycapronsäureestern die Bildung von Hochsiedern zu minimieren und die Ausbeute und Selektivität zu verbessern.

Diese Aufgabe wird gelöst in einem Verfahren zur Herstellung von 6-Hydroxycapronsäureestern durch Umsetzen von 5-Formylvaleriansäureestern mit molekularem Wasserstoff in Gegenwart von Hydrierkatalysatoren bei erhöhter Temperatur, dadurch gekennzeichnet, daß man eine Temperatur von bis 140°C einhält.

Das neue Verfahren hat den Vorteil, daß die Bildung von Hochsiedern, wie di-, tri- und polymeren Hydroxycapronsäureestern wesentlich vermindert wird. Weiter hat das neue Verfahren den Vorteil, daß keine großen Mengen an Lösungsmittel wiedergewonnen und gereinigt werden müssen. Ferner hat das neue Verfahren den Vorteil, daß es in kurzer Zeit verläuft.

Bevorzugte 5-Formylvaleriansäureester leiten sich von Alkanolen mit 1 bis 12 Kohlenstoffatomen, Cycloalkanolen mit 5 bis 7 Kohlenstoffatomen Aralkanolen mit 7 oder 8 Kohlenstoffatomen oder Phenolen mit 6 bis 8 Kohlenstoffatomen ab. Besonders bevorzugte Ausgangsstoffe sind 5-Formylvaleriansäure C₁-C₄-Alkylester. Beispielsweise seien genannt 5-Formylvaleriansäuremethylester, 5-Formylvaleriansäureethylester, 5-Formylvaleriansäure-n-propylester, 5-Formylvaleriansäure-i-propylester, 5-Formylvaleriansäure-n-butylester, 5-Formylvaleriansäure-2-ethylhexylester, 5-Formylvaleriansäurecyclohexylester oder 5-Formylvaleriansäurephenylester.

Besondere technische Bedeutung hat 5-Formylvaleriansäuremethylester erlangt.

Bevorzugte Hydrierkatalysatoren sind Metalle der 8. Nebengruppe des Periodischen Systems wie Nickel, Cobalt oder Edelmetalle wie Ruthenium, Platin oder Palladium. Die Katalysatoren können aktivierende Zusätze wie Zirkon, Mangan, Kupfer oder Chrom enthalten. Die Katalysatoren können als Vollkatalysatoren oder auf Träger wie Aluminiumoxid, Kieselgel, Tonerde oder Aktivkohle aufgebracht verwendet werden.

Bevorzugt sind Katalysatoren, die mindestens eines der Metalle Nickel und Cobalt enthalten. Vorteilhaft verwendet man Raney-Nickel oder Raney-Cobalt. Andere bevorzugte Katalysatoren enthalten 80 bis 100 Gew.% Nickel und/oder Cobalt und bis zu 20 Gew.% aktivierende Metalle wie Kupfer und/oder Chrom. Besonders vorteilhaft werden solche Katalysatoren als Trägerkatalysatoren z.B. auf Kieselsäure, Silikaten, Aluminiumphosphat, Borphosphat, Aluminiumoxid oder Aktivkohle aufgebracht verwendet. Der Gehalt an katalytischen aktiven Metallen solcher Trägerkatalysatoren beträgt in der Regel 5 bis 80 Gew.%, bezogen auf die Summe von katalytisch aktiven Metallen und Träger. Im allgemeinen werden solche Trägerkatalysatoren als Stränge von z.B. 2 bis 4 mm Durchmesser und bis zu 10 mm Länge oder als Tabletten z.B. mit einem Durchmesser von 3 bis 5 mm angewandt.

Die Hydrierung führt man bei einer Temperatur von 40 bis 140°C, vorzugsweise 50 bis 130°C, insbesondere 60 bis 120°C durch. Sofern man die Umsetzung in der Gasphase durchführt, hat sich Atmosphärendruck bewährt. Vorteilhaft führt man die Umsetzung in flüssiger Phase unter erhöhtem Druck, z.B. von 10 bis 400 bar vorteilhaft von 20 bis 300 bar, insbesondere von 50 bis 250 bar durch.

Wasserstoff wird in der Regel im Überschuß z.B. von 1,1 bis 50 Mol, insbesondere von 2 bis 30 Mol je Mol 5-Formylvaleriansäureestern eingesetzt. Ferner hat es sich als vorteilhaft erwiesen, eine Verweilzeit von 10 bis 240 Min. insbesondere 10 bis 60 Min. bei der Hydrierung einzuhalten. Darüber hinaus hat sich eine Belastung von 0,2 bis 5 kg 5-Formylvaleriansäureester je Liter Katalysator und Stunde bewährt.

Die Umsetzung kann diskontinuierlich z.B. in einem Druckgefäß unter Durchmischung, vorteilhaft jedoch kontinuierlich in einer Reaktionszone mit festangeordneten Katalysatoren in der Gasphase, insbesondere jedoch in der Flüssigphase in Sumpf- oder Rieselfahrweise durchgeführt werden. Besonders bewährt hat es sich, wenn man während der Hydrierung eine Rückvermischung, z.B. durch richtige Wahl der Reaktorgeometrie und/oder der Verweilzeit, vermeidet.

Bei der Hydrierung können unter Reaktionsbedingungen inerte Lösungsmittel mitverwendet werden. Geeignete Lösungsmittel sind beispielsweise Alkohole wie Methanol oder Ethanol, Ether wie Tetrahydrofuran oder Dioxan. Falls Lösungsmittel mitverwendet werden, enthält der zu hydrierende 5-Formylvaleriansäureester vorteilhaft bis zu 50 Gew.%, insbesondere bis zu 80 Gew.% Lösungsmittel. Besonders bewährt hat es sich jedoch die Hydrierung ohne Mitverwendung von zusätzlichen Lösungsmitteln durchzuführen.

In einer bevorzugten Ausführungsform des erfindungsgemäßen Verfahrens wird der 5-Formylvaleriansäureester gegebenenfalls mit den angegebenen Mengen Lösungsmitteln zusammen mit Wasserstoff von unten nach oben durch einen fest angeordnetes Bett des Hydrierkatalysators geleitet, wobei man die angegebenen Temperaturen und Drücke einhält. Nach Abkühlen und Entspannen wird das so erhaltene Reaktionsgemisch durch Destillation aufgearbeitet.

Die nach dem erfindungsgemäßen Verfahren erhältlichen 6-Hydroxycapronsäureester eignen sich zur Herstellung von Caprolacton.

Das Verfahren nach der Erfindung sei an folgenden Beispielen veranschaulicht. Im folgenden bezeichnen 5-FVSE = 5-Formylvalariansäuremethylester, 6-HCE = 6-Hydroxycapronsäuremethylester und C10 = Caprolacton.

### Beispiel 1

150 g 5-FVSE, 40 g Methanol und 8 g Raney-Nickel wurden bei einer Temperatur von 100 bis 130°C unter einem Druck von 120 bis 280 bar Wasserstoff in einem 300 ml Rührautoklaven 4 Stunden gerührt. Nach Abkühlen und Entspannen wurde der Katalysator abfiltriert. Eine Analyse des flüssigen Reaktionsaustrages durch quantitative Gaschromatographie ergab die in Tabelle I angegebenen Werte.

**Tabelle 1**

| No. | Temp. [°C] | Druck [bar] | Umsatz [mol-%] | Ausb. an 6-HCE [mol-%] | Ausb. an CLO [mol-%] |
|---|---|---|---|---|---|
| 1 | 130 | 280 | > 99 | 95 | 1 |
| 2 | 130 | 200 | > 99 | 93 | 2 |
| 3 | 130 | 150 | > 99 | 95 | 1 |
| 4 | 130 | 120 | > 99 | 94 | 2 |
| 5 | 100 | 120 | 99 | 91 | 2 |

### Beispiel 2

400 g 5-FVSE, 2000 g Methanol und 40 g Raney-Nickel wurden bei 130°C unter einem Druck von 100 bar Wasserstoff in einem 5000 ml-Rollautoklav 7,5 Stunden umgesetzt. Anschließend wurde der Reaktionsaustrag über Kieselgur filtriert, das Lösungsmittel im Vakuum destilliert und der Rückstand im Vakuum fraktionierend destilliert. Die Ausbeute betrug 392 g (96 % d. Th.) reiner 6-Hydroxycapronsäuremethylester vom Sdp. 114°C/8 mbar

### Beispiel 3

150 g 5-FVSE und 8 g Raney-Nickel wurden bei 100°C unter einem Druck von 120 bar Wasserstoff in einem 300 ml Rührautoklav 4 Stunden gerührt. Nach 0,75, 1,5 und 4 Stunden erfolgte jeweils nach Abfiltrieren des Katalysators eine Analyse des Reaktionsgemisches durch quantitative Gaschromatographie. Die Ergebnisse sind aus Tabelle II zu entnehmen.

**Tabelle II**

| Versuchsdauer [h] | Umsatz [mol-%] | Ausb. an 6-HCE [mol-%] | Ausb. an CLO [mol-%] |
|---|---|---|---|
| 0,75 | 70 | 69 | < 1 |
| 1,50 | 83 | 82 | < 1 |
| 4,00 | > 99 | 95 | 2 |

### Beispiel 4

150 g 5-FVSE und der in Tabelle III angegebene Katalysator wurden bei 100°C unter einem Druck von 120 bar Wasserstoff in einem 300 ml Rührautoklaven 4 Stunden gerührt. Nach 4 Stunden wurde der Katalysator abfiltriert und das Reaktionsgemisch durch quantitative Gaschromatographie analysiert. Die Ergebnisse sind aus Tabelle 111 zu entnehmen.

**Tabelle III**

| No. | Katalysator | Umsatz [mol-%] | Ausb. an 6-HCE [mol-%] | Ausb. an CLO [mol-%] |
|---|---|---|---|---|
| 1 | 8 g Raney-Nickel | > 99 | 95 | 2 |
| | 8 g Raney-Cobalt | 99 | 94 | 2 |

### Beispiel 5

Ein vertikaler Rohrreaktor (Durchmesser 16 mm, Füllhöhe 25 cm, ölbeheizter Doppelmantel) wurde mit 50 ml eines Nickel-Katalysators (80 Gew.% Nickeloxid in feiner Verteilung auf Aluminiumoxid; 1,5 mm Stänge) gefüllt. Der Katalysator wurde in einem Zeitraum von 18 Stunden unter schrittweisem Erhöhen der Temperatur von 60 auf 330°C unter Steigern des Wasserstoffgehalts in dem zur Reduktion benutzten Stickstoff-Wasserstoff-Gemisch (anfangs 5 Vol .%, gegen Ende 50 Vol .% Wasserstoff) reduziert.

Danach wurden unter gleichzeitigem Durchleiten von Wasserstoff (Molverhältnis 5-FVSE : H₂ = 1 : 30) bei 85°c unter einem Druck von 100 bar stündlich von unten nach oben 50 g 5-FVSE durch den Reaktor gepumpt (Verweilzeit ca. 50 Minuten). Vom Kopf des Reaktors gelangte das Reaktionsgemisch über einen Kühler in einen Abscheider, aus dem das flüssige Reaktionsprodukt und das Abgas getrennt ausgeschleust wurden. Das flüssige Reaktionsprodukt enthielt nach quantitativer gaschromatographischer Analyse 94,5 Gew.% 6-HCE, 2,2 Gew.% CLO und 0,3 Gew.% 5-FVSE. Das flüssige Reaktionsprodukt wurde über eine Versuchszeit von 8 Stunden gesammelt und im Vakuum fraktionierend destilliert. Die Ausbeute betrug 447 g (92 % d. Th. bez. 5-FVSE) reiner 6-Hydroxycapronsäuremethylester vom Sdp. 62°C/0,2 mbar.

### Vergleichsbeispiel

Unter sonst gleichen Bedingungen wie im Beispiel 5) jedoch bei einer Temperatur von 150°C und einer Verweilzeit von ca. 100 Minuten wurde ein flüssiger Reaktionsaustrag erhalten, der nach quantitativer gaschromatographischer Analyse 75,6 Gew.% 6-HCE, 2,6 Gew.% CLO und 1,5 Gew.% 5-FVSE enthielt. Die relativ hohe Reaktionstemperatur bewirkt zusammen mit der längeren Verweilzeit eine deutliche Zunahme der Bildung von Hochsiedern und damit eine drastische Abnahme der Selektivität.

## Patentansprüche

1. Verfahren zur Herstellung von 6-Hydroxycapronsäureestern durch Umsetzen von 5-Formylvaleriansäureestern mit molekularem Wasserstoff in Gegenwart von Hydrierkatalysatoren bei erhöhter Temperatur, dadurch gekennzeichnet, daß man die Umsetzung bei einer Temperatur von 40 bis 140°C durchführt.

2. Verfahren nach Anspruch 1, dadurch gekennzeichnet, daß man Nickel und/oder Kobalt enthaltende Katalysatoren verwendet.

3. Verfahren nach den Ansprüchen 1 und 2, dadurch gekennzeichnet, daß man Raney-Nickel und/oder Raney-Kobalt verwendet.

4. Verfahren nach den Ansprüchen 1 bis 3, dadurch gekennzeichnet, daß man eine Verweilzeit von 10 bis 60 Minuten einhält.

5. Verfahren nach den Ansprüchen 1 bis 4, dadurch gekennzeichnet, daß man eine Temperatur von 50 bis 130°C einhält.

6. Verfahren nach den Ansprüchen 1 bis 5, dadurch gekennzeichnet, daß man einen Druck von 20 bis 300 bar einhält.

7. Verfahren nach den Ansprüchen 1 bis 6, dadurch gekennzeichnet, daß man die Hydrierung im wesentlichen ohne Rückvermischung durchführt.

8. Verfahren nach den Ansprüchen 1 bis 7, dadurch gekennzeichnet, daß man 5-Formylvalariansäure C₁-C₄-Alkylester als Ausgangsstoffe verwendet.

## Claims

1. A process for the preparation of a 6-hydroxycaproic ester by reacting a 5-formylvaleric ester with molecular hydrogen in the presence of a hydrogenation catalyst at elevated temperatures which comprises carrying out the reaction at from 40 to 140°C.

2. A process as claimed in claim 1, wherein catalysts containing nickel and/or cobalt are used.

3. A process as claimed in claims 1 and 2, wherein Raney nickel and/or Raney cobalt are used.

4. A process as claimed in any of claims 1 to 3, wherein a residence time of from 10 to 60 minutes is maintained.

5. A process as claimed in any of claims 1 to 4, wherein a temperature of from 50 to 130°C is maintained.

6. A process as claimed in any of claims 1 to 5, wherein a pressure of from 20 to 300 bar is maintained.

7. A process as claimed in any of claims 1 to 6, wherein the hydrogenation is carried out essentially without backmixing.

8. A process as claimed in any of claims 1 to 7, wherein a C₁-C₄-alkyl 5-formylvalerate is used as the starting material.

## Revendications

1. Procédé de préparation d'esters d'acide 6-hydroxycaproïque par réaction d'esters d'acide 5-formylvalérique avec de l'hydrogène moléculaire en présence de catalyseurs d'hydrogénation à température élevée, caractérisé en ce qu'on conduit la réaction à une température de 40 à 140°C.

2. Procédé selon la revendication 1, caractérisé en ce qu'on utilise des catalyseurs contenant du nickel et/ou du cobalt.

3. Procédé selon la revendication 1 ou 2, caractérisé en ce qu'on utilise du nickel de Raney et/ou du cobalt de Raney.

4. Procédé selon l'une quelconque des revendications 1 à 3, caractérisé en ce qu'on observe une durée de séjour de 10 à 60 mn.

5. Procédé selon l'une quelconque des revendications 1 à 4, caractérisé en ce qu'on observe une température de 50 à 130°C.

6. Procédé selon l'une quelconque des revendications 1 à 5, caractérisé en ce qu'on observe une pression de 20 à 300 bar.

7. Procédé selon l'une quelconque des revendications 1 à 6, caractérisé en ce qu'on conduit l'hydrogénation essentiellement sans remélangeage.

8. Procédé selon l'une quelconque des revendications 1 à 7, caractérisé en ce qu'on utilise du 5-formylvalérate d'alkyle en C₁-C₄ comme substance de départ.
